# EUROPÄISCHE PATENTSCHRIFT

(11) **EP 0 871 413 B1**
(45) Veröffentlichungstag und Bekanntmachung des Hinweises auf die Patenterteilung: **22.08.2001**
(21) Anmeldenummer: 96942165.0
(22) Anmeldetag: 09.12.1996
(51) Int. Cl.: A61F 2/06

(54) **EINRICHTUNG ZUM STABILISIEREN VON ANGIOPLASTISCH BEHANDELTEN TEILBEREICHEN EINER GEFÄSSWAND (STENT)**
DEVICE FOR STABILISING ANGIOPLASTICALLY TREATED PARTIAL REGIONS OF A VESSEL WALL (STENT)
DISPOSITIF POUR STABILISER DES ZONES PARTIELLES D'UNE PAROI VASCULAIRE TRAITEES PAR ANGIOPLASTIE (EXTENSEUR)

(30) Priorität: 11.12.1995 AT 67195 U
(43) Veröffentlichungstag der Anmeldung: 21.10.1998
(73) Patentinhaber: Hassan, Ali, 1210 Wien (AT); Glogar, Helmut Dietmar, 1010 Wien (AT)
(72) Erfinder: Hassan, Ali, 1210 Wien (AT); Glogar, Helmut Dietmar, 1010 Wien (AT)
(74) Vertreter: Haffner, Thomas M., Dr.
(86) Internationale Anmeldenummer: AT9600243
(87) Internationale Veröffentlichungsnummer: WO9721399

(56) Entgegenhaltungen:
- EP-A- 0 421 729
- US-A- 5 314 472

## Beschreibung

Die Erfindung bezieht sich auf eine Einrichtung zum Stabilisieren von stenosierten bzw. angioplastisch behandelten Teilbereichen einer Gefäßwand, bestehend aus einem aufweitbaren Stützteil als Stent, welcher als rohrförmiger Körper ausgebildet ist, wobei die Wandung aus wenigstens einem in Umfangsrichtung mäanderförmig oder zick-zackförmig geformten Tragelement besteht, dessen oder deren Flächen- bzw. Windungsdichte bei gleichem Material in axialer Richtung des Stents unterschiedlich ist, und daß das oder die Tragelemente in Bereichen mit verschiedener Flächen- bzw. Windungsdichte bei auf im wesentlichen über die axiale Länge gleichem aufgeweiteten Durchmesser unterschiedliche radiale Druckkräfte aufweisen.

Intraluminale Gefäßprothesen, wie sie üblicherweise als Stent bezeichnet werden, können Plaqueanteile aus dem Gefäßlumen fernhalten und haben eine gefäßwandstützende Wirkung. Somit wird ein hämodynamisch adäquates Gefäßlumen auf lange Sicht gewährleistet. Die Gefäßwand selbst ist in der Regel elastisch ausgebildet und die aufzuwendenden Stützkräfte müssen jeweilige Rückstellkräfte der elastischen Gefäßwand antagonisieren.

Die Ballonangioplastie hat sich zu einer sehr wirkungsvollen und wenig belastenden Therapie bei gefährlich verengten Herzkranzgefäßen entwickelt. Die Hauptlimitation des ansonsten eleganten Verfahrens ist die hohe Anzahl an Rezidivstenosen: Bei jedem dritten Patienten tritt die Engstelle in den ersten 3-6 Monaten nach dem Eingriff wieder auf; die Konsequenzen sind von großer klinischer und gesundheitsökonomischer Relevanz.

Die Implantation von intraluminalen gefäßwandstützenden Prothesen hat sich als besonders erfolgreich bewährt. Der wesentliche Vorteil der Implantation eines Stents liegt in der signifikant niedrigeren Restenoserate gegenüber der Ballondilatation (Benestent-I-Studie: 22% Restenoserate), sowie in den durch moderne Antikoagulationsschemata und Beschichtung mit verschiedenen Pharmaka reduzierten Komplikationen nach der Implantation (Benestent-II-Studie).

Die Stents variieren hinsichtlich ihrer Materialien, Designs und Dimensionen. Bezüglich des Implantationsmechanismus unterscheidet man selbstentfaltende (self-expandable) und ballonentfaltbare (balloon-expandable) Stents. Der an einem Ballon-Katheter (delivery system) fixierte Stent wird über eine Führungsspirale in das stenosierte Gefäßlumen eingeführt, dann wird der Implantationsmechanismus ausgelöst (im Falle eines "balloon-expandable" Stents wird der Ballon mit Hilfe eines Fluiddrucksystems für eine gegebene Zeitspanne im Sekundenbereich aufgebläht), anschließend wird das "Delivery System" aus dem Gefäßsegment entfernt, während der Stent seine Funktion als lebenslange intraluminale Gefäßprothese wahrnimmt.

Eine bekannte Konstruktion eines Stents ergibt sich zum Beispiel aus der US-PS 5,383,892, bei welcher zumindest ein flexibler elastisch aufweitbarer, zylindrischer Teilbereich und starre, plastisch aufweitbare zylindrische Teil- bzw. Endbereiche aus verschiedenen Materialien abwechselnd miteinander verbunden sind.

Aus der US-A 5314472 ist eine Gefäßprothese bekannt geworden, welche aus einem in Wellenform gebogenen Draht aufgebaut ist. Die Gefäßprothese weist einen Mittelteil und zwei Endteile auf, wobei die Windungsdichte des Drahtes in den Endteilen höher als im Mittelteil ist, womit eine gute Befestigung der Gefäßprothese im Gefäß erzielt wird. Die höhere Windungsdichte in den Endteilen wird dabei durch eine geringere Amplitude der Windungen des wellenförmig gebogenen Drahtes erreicht.

Gegenwärtig bekannte Stents sind jedoch für die Behandlung von komplexen bzw. langstreckigen Stenosen nicht optimal geeignet, da technische Schwierigkeiten bei der Stentplazierüng durch erhöhte Inflexibilität bestehen, sowie höhere Restenoserate beobachtet werden. Während der Stentimplantation .wird die Orientierung nur durch die Angiographie gewährleistet, wobei diese Plazierung durch den Herzschlag erschwert ist. Unter erschwerenden Bedingungen kommt es relativ oft zur Implantation mehrerer Stents zur Revaskularisation einer komplexen bzw. langen Läsion, was mit relativ hohen Restenoseraten assoziiert ist, da der mechanische und thrombogene Zustand innerhalb solcher Stentketten nicht gesichert ist. Weiters werden durch multiple Stentimplantation angrenzende "normale" Gefäßstrukturen einem inadäquaten mechanischen und thrombogenen Trauma ausgesetzt.

Die Erfindung zielt nun darauf ab, eine Stenteinrichtung der eingangs genannt Art dahingehend weiterzubilden, daß eine solche Gefäßprothese sowohl einfache als auch komplexe bzw. langstreckige Stenosen adäquat abdecken kann, wobei eine gleichzeitige Anpassung an die longitudinale Abdeckstrecke und Minimierung des mechanischen, thrombogenen und wie auch immer gearteten, für die Revaskularisation mit Hilfe der Stentimplantation unumgänglich erscheinenden Traumas an die angrenzenden "normalen" Gefäßstrukturen erzielt wird.

Zur Lösung dieser Aufgabe besteht die erfindungsgemäße Einrichtung im wesentlichen darin, daß die mittleren Bereiche eine höhere Flächen- bzw. Windungsdichte und in aufgeweitetem Zustand höhere Druckfestigkeit aufweisen als die endständigen Bereiche und daß in axialer Richtung gestaffelte Bereiche des Stents mit unterschiedlich gekrümmten oder einen unterschiedlichen Windungsabstand aufweisenden Tragelementen vorgesehen sind und daß das oder die Tragelemente von stabförmigem gewundenen Material mit über die axiale Länge des Stents gleichbleibendem Materialdurchmesser und/oder Materialquerschnitt gebildet sind. Dadurch, daß die mittleren Bereiche eine höhere Flächen- bzw. Windungsdichte und in aufgeweitetem Zustand höhere Druckfestigkeit aufweisen als die endständigen Bereiche werden die über die axiale Länge unterschiedlich starken Kräfte der elastischen Gefäßwand in geeigneter Weise antagonisiert, womit es möglich ist, den Stent an die jeweiligen Bedürfnisse optimal anzupassen. Dadurch, daß in axialer Richtung gestaffelte Bereiche des Stents mit unterschiedlich gekrümmten oder einen unterschiedlichen Windungsabstand aufweisenden Tragelementen vorgesehen sind und daß das oder die Tragelemente von stabförmigem gewundenen Material mit über die axiale Länge des Stents gleichbleibendem Materialdurchmesser und/oder Materialquerschnitt gebildet sind, wird die Manövrierbarkeit und damit die Sicherheit der Positionierung an der gewünschten Stelle wesentlich verbessert. Es können wesentlich längere Stents sicher eingebracht werden, wodurch gegenüber der üblichen Verwendung von mehreren sequentiell implantierten Stents (Stentketten) mit einem einzigen über einen langen Läsionsbereich wirksamen Teilbereich der Gefäßwand die gewünschte Stützkraft sichergestellt werden kann. Es gelingt somit insgesamt eine wesentlich einfachere und sichere Plazierung, während die Gefahr einer Dislokation und damit einer Komplikation wird verringert wird.

Dabei kann der Stent aus einem einzigen langgestreckten Tragelement, beispielsweise einem Draht oder aus einer Mehrzahl von Tragelementen aufgebaut sein, wobei diese Ausbildung mit Vorteil so getroffen ist, daß eine Mehrzahl von Tragelementen in axialer Richtung gelenkig miteinander verbunden ist.

Ein aus einem einzigen Draht aufgebauter Stent kann in axialer Richtung in Bereiche mit unterschiedlichen radialen Druckkräften unterteilt sein. Bei einem Stent mit einer Mehrzahl von miteinander verbundenen Tragelementen bestimmen diese jeweils unterschiedliche radiale Druckkräfte. Mit Vorteil ist die Ausbildung so getroffen, daß eine Mehrzahl von unterschiedlich geformten bzw. gewundenen Teilbereichen über jeweils ein sich in axialer Richtung erstreckendes Verbindungsglied verbunden sind. Dadurch ist eine hohe Flexibilität zwischen den einzelnen Tragelementen und somit eine gute Manövrierfähigkeit beim Einbringen des Stents gewährleistet.

In besonders vorteilhafter Weise ist eine Mehrzahl von Tragelementen koaxial und in radialer Richtung aufeinanderfolgend angeordnet. Es können so auch durch die Kombination von Tragelementen, die koaxial ineinandergeschoben werden, unterschiedliche radiale Druckkräfte ausgeübt werden.

In einer besonders bevorzugten Weise ist die Ausbildung so getroffen, daß das Material der Stützelemente mit pharmakologisch wirksamen Substanzen und/oder radioaktiven Isotopen dotiert ist. Durch die in axialer Richtung unterschiedlichen Flächen- bzw. Windungsdichten ist auch die Dotierungsmenge mit pharmakologisch wirksamen Substanzen oder radioaktiven Isotopen im gleichen Sinne unterschiedlich, und es kann die Dotierung in bestimmten Bereichen entsprechend der Flächen-bzw. Windungsdicke konzentriert werden.

In besonders vorteilhafter Weise wird für das Einbringen derartiger Stents so vorgegangen, daß der Stent zur Plazierung auf einen an sich gekrümmten Angioplastie-Ballon-Katheter mit in axialer Richtung verschieden steif ausgebildeter Ballonwand aufgeschoben ist, wodurch die erforderliche Aufweitung auch bei unterschiedlicher radialer Rückstellkraft des Stents zu einem im wesentlichen rohrförmigen Stützteil gelingt. Alternativ kann in besonders einfacher Weise die Anwendung so gestaltet werden, daß der Stent in einem an einer vorbestimmten Stelle abnehmbaren Hüllschlauch angeordnet ist.

Die Erfindung wird nachfolgend anhand von in der Zeichnung schematisch dargestellten Ausführungsbeispielen näher erläutert.

In dieser zeigen Fig.1 einen Stent, der aus einem einzigen Draht aufgebaut ist, Fig.2 einen Stent, der aus mehreren Tragelementen besteht und Fig.3 einen Stent, der in der Mitte aus einem einzigen Draht aufgebaut ist und an den Enden mit weiteren Tragelementen verbunden ist.

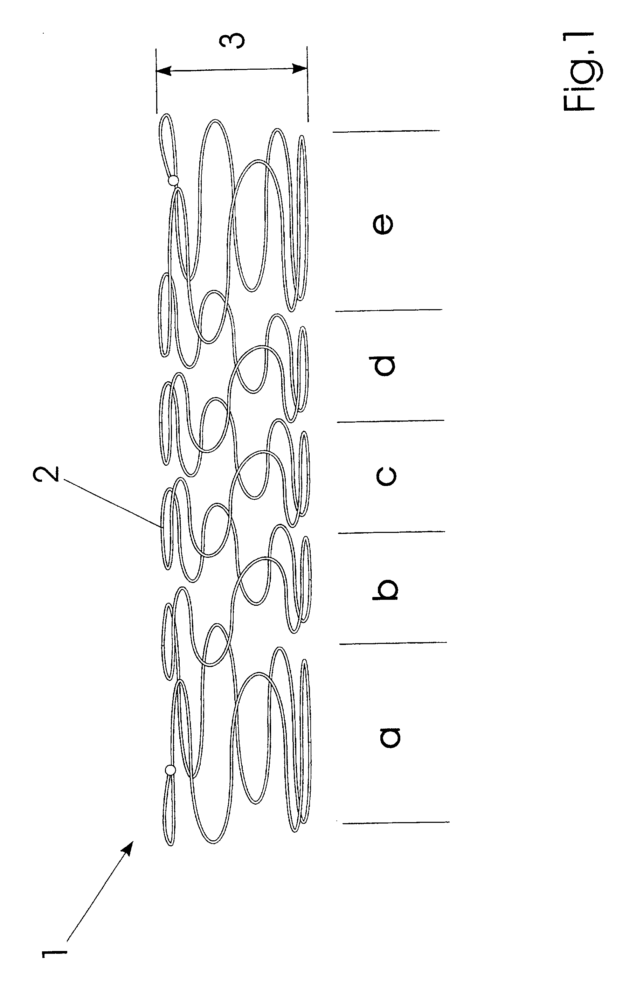

Der in Fig.1 mit 1 bezeichnete Stent weist in axialer Richtung aneinander anschließende Teilbereiche a bis e auf. Die Teilbereiche a und e zeichnen sich durch eine relativ geringe Flächen- bzw. Windungsdichte des Stützdrahtes 2 aus, sodaß in diesem Teilbereich nach dem Aufweiten auf den gewünschten Durchmesser 3 eine vorbestimmte Rückstellkraft bzw. Stützkraft erzielt wird. In den zwischen den endständigen Bereichen a und e liegenden Teilbereichen b, c und d, welche in axialer Richtung aneinander anschließen, ist der Stützdraht 2 zu engeren Maschen gewickelt, wodurch eine höhere Steifheit auch in aufgeweitetem Zustand gewährleistet ist. Wenn in diesen Bereichen b, c und d in der Folge eine höhere elastische Rückstellkraft der Gefäßwand auftreten sollte, kann diese Rückstellkraft durch die Stützkraft der Drahtschleifen in den Bereichen b, c und d sicher aufgenommen werden, wohingegen in den endständigen Bereichen a und e geringere Stützkräfte ausgeübt werden. Insgesamt ergibt sich somit eine selektive Anpassung der jeweils erforderlichen Stützkräfte an den Verlauf, und die Art der Läsion der Gefäßwand, wobei durch die Unterteilung des Stents in axialer Richtung aneinander anschließende Abschnitte a bis e mit unterschiedlichen radialen Rückstellkräften gleichzeitig die Manövrierbarkeit des Stents wesentlich verbessert wird.

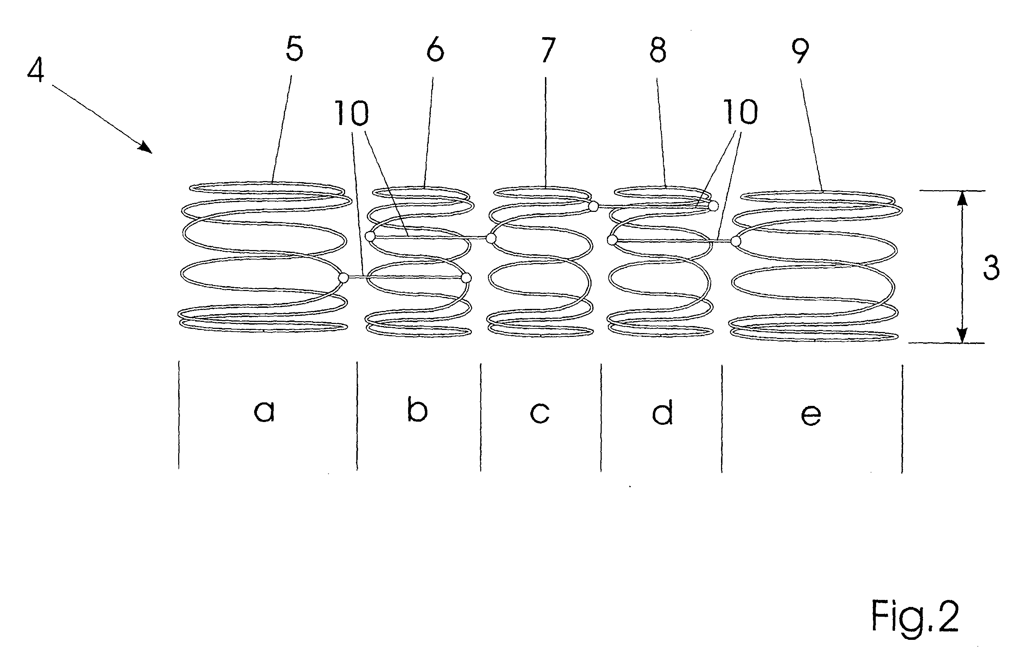

Bei der Ausbildung nach Fig.2 ist ein Stent 4 mit mehreren Tragelementen 5-9 dargestellt, welcher analog zu dem in Fig.1 beschriebenen Stent in Teilbereiche a bis e gegliedert ist. Bei diesem Stent sind die Teilbereiche a und e durch relativ weitmaschige Tragelemente 5 und 9 und die Teilbereiche b, c und d durch relativ engmaschige Tragelemente 6, 7 und 8 gegeben, wodurch sich wiederum eine höhere elastische Rückstellkraft in den inneren Teilbereichen b, c und d und eine geringere Stützkraft in den endständigen Bereichen ergibt. Die Tragelemente 5 bis 9 sind untereinander jeweils mit einem Verbindungstück 10 verbunden.

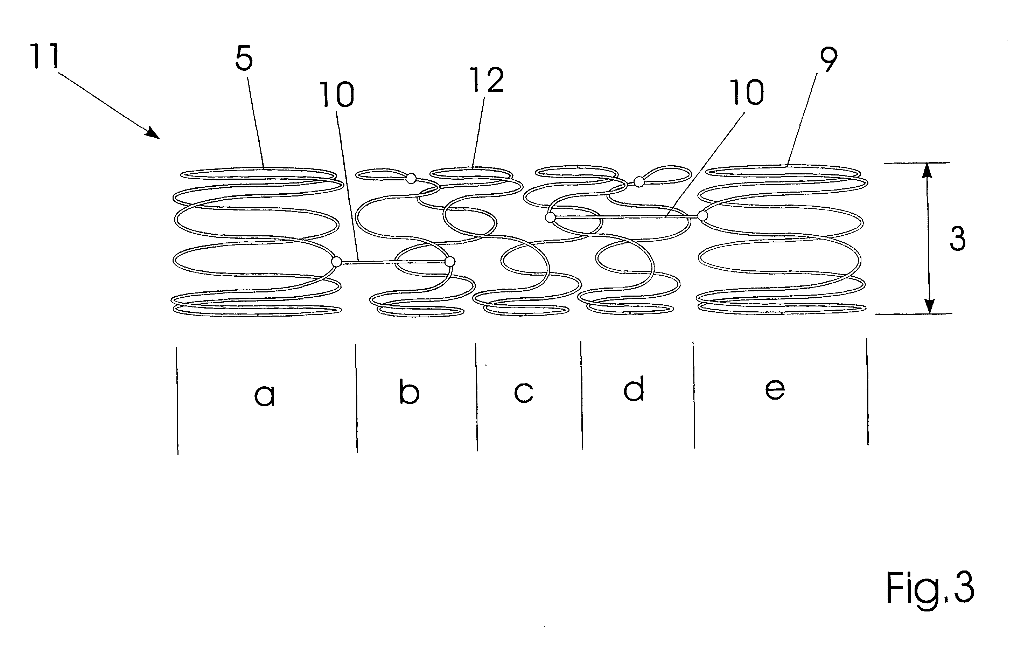

Fig.3 zeigt einen Stent 11, der eine Kombination von den in Fig.1 und 2 beschriebenen Stents 1 und 4 darstellt. Auch dieser Stent ist wie die vorhergehenden in Teilbereiche a bis e gegliedert, wobei ein aus einem einzigen Draht 12 bestehender engmaschiger Teilbereich b, c und d mit weitmaschigen Teilbereichen a und e, die aus den Tragelementen 5 und 9 bestehen, mit je einem Verbindungsstück 10 verbunden sind. Es ergeben sich wiederum höhere Rückstellkräfte in den Teilbereichen b, c und d als in den endständigen Teilbereichen a und e.

Die einzelnen beschriebenen Stents unterscheiden sich bezüglich ihren Eigenschaften, wobei der Stent 1 in Fig.1 eine ausgezeichnete Manövrierfähigkeit und Anpassung an die Bewegung der Blutgefäße aufweist, der Stent 4 in Fig.2 eine minimale Verkürzung beim Aufweiten zeigt und der Stent 11 in Fig.3 eine sehr gute Eckenstabilität aufweist. Bei einer Dotierung mit pharmakologisch wirksamen bzw. radioaktiven Substanzen ist die Konzentration bzw. Dosis in den mittleren Bereichen b, c, d höher als in den endständigen Bereichen a und e.

## Patentansprüche

1. Einrichtung zum Stabilisieren von stenosierten bzw. angioplastisch behandelten Teilbereichen einer Gefäßwand, bestehend aus einem aufweitbaren Stützteil als Stent (1,4,11), welcher . als rohrförmiger Körper ausgebildet ist, wobei die Wandung aus wenigstens einem in Umfangsrichtung mäanderförmig oder zick-zackförmig geformten Tragelement (2,5,6,7,8,9,12) besteht, dessen oder deren Flächen- bzw. Windungsdichte bei gleichem Material in axialer Richtung des Stents unterschiedlich ist, und daß das oder die Tragelemente in Bereichen (a,b,c,d,e) mit verschiedener Flächen- bzw. Windungsdichte bei auf im wesentlichen über die axiale Länge gleichem aufgeweiteten Durchmesser (3) unterschiedliche radiale Druckkräfte aufweisen, dadurch gekennzeichnet, daß die mittleren Bereiche eine höhere Flächen- bzw. Windungsdichte und in aufgeweitetem Zustand höhere Druckfestigkeit aufweisen als die endständigen Bereiche und daß in axialer Richtung gestaffelte Bereiche (a, b, c, d, e) des Stents (1,4,11) mit unterschiedlich gekrümmten oder einen unterschiedlichen Windungsabstand aufweisenden Tragelementen (2,5,6,7,8,9,12) vorgesehen sind und daß das oder die Tragelemente (2,5,6,7,8,9,12) von stabförmigem gewundenen Material mit über die axiale Länge des Stents (1,4,11) gleichbleibendem Materialdurchmesser und/oder Materialquerschnitt gebildet sind.

2. Einrichtung nach Anspruch 1, dadurch gekennzeichnet, daß eine Mehrzahl von Tragelementen (5,6,7,8,9,12) in axialer Richtung gelenkig miteinander verbunden ist.

3. Einrichtung nach Anspruch 1 oder 2, dadurch gekennzeichnet, daß eine Mehrzahl von unterschiedlich geformten bzw. gewundenen Teilbereichen (5,6,7,8,9,12) über jeweils ein sich in axialer Richtung erstreckendes Verbindungsglied (10) verbunden ist.

4. Einrichtung nach einem der Ansprüche 1,2 oder 3, dadurch gekennzeichnet, daß eine Mehrzahl von Tragelementen koaxial und in radialer Richtung aufeinanderfolgend angeordnet ist.

5. Einrichtung nach einem der Ansprüche 1 bis 4, dadurch gekennzeichnet, daß das Material der Stützelemente (2,5,6,7,8, 9,12) mit pharmakologisch wirksamen Substanzen und/oder radioaktiven Isotopen dotiert ist.

6. Einrichtung nach einem der Ansprüche 1 bis 5, dadurch gekennzeichnet, daß der Stent (1,4,11) zur Plazierung auf einen an sich bekannten Angioplastie-Ballon-Katheter mit in axialer Richtung verschieden steif ausgebildeter Ballonwand aufgeschoben ist.

## Claims

1. A device for stabilizing stenosed or angioplastically treated partial regions of a vessel wall, comprised of an expandable supporting part constituting a stent (1, 4, 11) and designed as a tubular body, wherein the wall is formed by at least one supporting element (2,5,6,7,8,9,12) which is meander-shaped or zig-zag-shaped in the peripheral direction and whose surface or coil density/densities differ(s) in the axial direction of the stent using the same material, and wherein the supporting element(s) in regions (a,b,c,d,e) of different surface or coil densities exhibit different radial compressive forces at a diameter (3) substantially equally expanded over the axial length, characterized in that the central regions exhibit higher surface or coil densities and, in the expanded state, higher compressive strengths than the end regions, and that regions (a,b,c,d,e) of the stent (1,4,11) which are staggered in the axial direction are provided including supporting elements (2,5,6,7,8,9,12) that are differently curved or have different coil distances, and that the supporting element(s) (2,5,6,7,8,9,12) is/are comprised of a rod-shaped and coiled material having a constant material diameter and/or material cross section over the axial length of the stent (1,4,11).

2. A device according to claim 1, characterized in that a plurality of supporting elements (5,6,7,8,9,12) are articulately connected in the axial direction.

3. A device according to claim 1 or 2, characterized in that a plurality of differently shaped or coiled partial regions (5,6,7,8,9,12) are each connected by a connecting member (10) extending in the axial direction.

4. A device according to any one of claim 1, 2 or 3, characterized in that a plurality of supporting elements are arranged coaxial, following one another in the radial direction.

5. A device according to any one of claims 1 to 4, characterized in that the material of the supporting elements (2,5,6,7,8,9,12) is doped with pharmacologically active substances and/or radioactive isotopes.

6. A device according to any one of claims 1 to 5, characterized in that the stent (1,4,11) is slipped on to be placed on an angioplasty balloon catheter known per se and having a balloon wall made to be differently stiff in the axial direction.

## Revendications

1. Dispositif pour stabiliser des zones partielles, sténosées ou traitées par angioplastie, d'une paroi vasculaire, constitué d'une pièce d'appui élargissable servant de stent (1, 4, 11), qui est configurée comme un corps tubulaire, la paroi étant constituée d'au moins un élément porteur (2, 5, 6, 7, 8, 9, 12), ayant dans la direction périphérique la forme de méandres ou de zigzags, éléments porteurs dont la densité superficielle ou la densité des spires varie, pour un seul et même matériau, dans la direction axiale du stent, et en ce que le ou les éléments porteurs présentent dans des zones (a, b, c, d, e) ayant différentes densités superficielles ou de spires, et pour un diamètre (3) élargi, essentiellement constant sur toute la longueur axiale, des forces de compression radiale différentes, caractérisé en ce que les zones centrales présentent une densité superficielle ou une densité des spires plus grande, et à l'état élargi une résistance à la compression plus grande, que les zones terminales, et que l'on prévoit des zones étagées dans la direction axiale (a, b, c, d, e) du stent (1, 4, 11), comportant des éléments porteurs (2, 5, 6, 7, 8, 9, 12) présentant des courbures différentes ou des écarts différents entre spires, et que le ou les éléments porteurs (2, 5, 6, 7, 8, 9, 12) sont formés d'un matériau spiralé en forme de barre, ayant sur la longueur axiale du stent (1, 4, 11) un diamètre et/ou une section transversale constants du matériau.

2. Dispositif selon la revendication 1, caractérisé en ce qu'un grand nombre d'éléments porteurs (5, 6, 7, 8, 9, 12) sont reliés l'un à l'autre d'une manière articulée dans la direction axiale.

3. Dispositif selon la revendication 1 ou 2, caractérisé en ce qu'un grand nombre de zones partielles (5, 6, 7, 8, 9, 12), ayant des formes ou des structures de spires différentes, sont reliées par un organe de liaison (10), qui s'étend dans la direction axiale.

4. Dispositif selon l'une des revendications 1, 2 ou 3, caractérisé en ce qu'un grand nombre d'éléments porteurs sont disposés successivement d'une manière coaxiale et dans la direction radiale.

5. Dispositif selon l'une des revendications 1 à 4, caractérisé en ce que le matériau des éléments porteurs (2, 5, 6, 7, 8, 9, 12) est dopé par des substances à effet pharmacologique et/ou des isotopes radioactifs.

6. Dispositif selon l'une des revendications 1 à 5, caractérisé en ce que le stent (1, 4, 11) est, pour mise en place sur une sonde à ballonnet pour angioplastie connue en soi, enfilé dans une paroi de ballonnet dont la rigidité varie dans la direction axiale.
